# EUROPEAN PATENT SPECIFICATION

(11) **EP 1 732 637 B1**
(45) Date of publication and mention of the grant of the patent: **05.08.2009**
(21) Application number: 05705330.8
(22) Date of filing: 07.01.2005
(51) Int. Cl.: A61M 31/00, A61M 37/00, A61M 25/00, A61M 25/10, A61M 25/09

(54) **MULTILUMEN CATHETERS**
MEHRLUMIGE KATHETER
CATHETERS MULTILUMIERE

(30) Priority: 09.01.2004 US 535669 P
(43) Date of publication of application: 20.12.2006
(73) Proprietor: Corazon Technologies, Inc., Menlo Park, CA 94025 (US)
(72) Inventor: CONSTANTZ, Brent R., Cupertino, California 95014 (US)
(74) Representative: Duxbury, Stephen
(86) International application number: PCT/US2005/000616
(87) International publication number: WO 2005/070109

(56) References cited:
- WO-A-01/15767
- WO-A-98/47558
- WO-A-99/58174
- US-A1- 2002 052 638
- US-B1- 6 592 544

## Description

### INTRODUCTION

### Field of the Invention

The field of this invention is atherosclerosis and related vascular conditions, and particularly catheter devices used for treating such conditions.

### Background of the Invention

The formation of plaques or lesions (atherosclerotic plaques or lesions), on vascular tissue, such as the inner surface of blood vessels, aortic valves, etc., is a major component of various vascular disease conditions. For example, plaques on heart related vascular structures, e.g., coronary artery intima, heart valves, etc., are often implicated in various heart disease conditions. Likewise, plaques or lesions present on the intima of peripheral vessels, e.g., arteries, are often implicated in various peripheral vascular disease conditions.

A variety of different protocols have been developed for treating diseases associated with the presence of vascular lesions or plaques. Such treatment methodologies generally involve mechanical removal or reduction of the lesion, and include: bypass surgery, balloon angioplasty, mechanical debridement, atherectomy, valve replacement, and the like. Despite the plethora of different treatment strategies that have been developed for the treatment of such vascular disease conditions, there are disadvantages associated with each technique, such as tissue damage, invasiveness, etc. For example, restenosis is a common complication that results in arteries in which lesions have been mechanically removed.

WO 99/58174 discloses a catheter that is used for positioning an expendable shunt device. The catheter comprises five lumens which are directed to several purposes: a perfusion lumen for transportation of a fluid, two lumens for monitoring pressure, a lumen for a guide wire and a lumen for balloon inflation. The lumens are in a non-coaxial position to each other. All lumens are connected by a luer type connector except for the lumen for the guide wire which is connected to a guide wire port.

US 2002/052638 discloses a catheter comprising a tubular body having a braid or a coil of metal. The catheter is adapted for balloon angioplasty or atherectomy. A cross-section of the catheter shaft shows a main lumen used for a therapeutic balloon catheter and a perfusion balloon catheter. At its periphery, there is a lumen for irrigation, an aspiration lumen, a lumen for a balloon on a wire catheter, an inflation lumen, and a not further specified lumen. The manifold has corresponding entrances for the lumens.

As such, there is continued interest in the development of new treatment protocols for the removal of vascular lesions from vascular tissue, as well as catheter devices that are used in such protocols.

### Literature

U.S. Patents of interest include: 4,329,994; 4,838,881; 5,149,330; 5,167,623; 5,207,648; 5,542,937; 6,004,310; and 6,013,068. Also of interest are U.S. Patent Nos.: 4,445,892; 4,573,966; 4,610,662; 4,636,195; 4,655,746; 4,824,436; 4,911,163; 4,976,733; 5,059,178; 5,090,960; 5,167,628; 5,195,955; 5,222,941; 5,380,284; 5,443,446; and 5,462,529. See also: WO 00/03651; WO 01/13985; WO 01/15767; WO 01/39783; WO 01/70320; and WO 02/15958.

### SUMMARY OF THE INVENTION

Multilumen fluid delivery systems are provided. The subject multilumen fluid delivery systems include a multilumen aspiration catheter over a guidewire, which in many embodiments is a hollow guidewire. Also provided are kits comprising various components of the subject systems. The subject multilumen fluid delivery systems and kits find use in a variety of different applications in which it is desired to flush a vascular site with at least one and preferably two different fluids, where particular applications of interest include the treatment of vascular lesions, and particularly coronary vascular lesions.

### BRIEF DESCRIPTION OF THE FIGURES

Each of the following figures provides examples diagrammatically illustrating aspects of the present invention.

Figs. 1A and 1B each provide a representation of an aspiration catheter according to the subject invention.

Figs. 2A and 2B each provide a representation of a guidewire element according to one embodiment of the subject invention, where the guidewire element is a hollow guidewire having an occlusive element at its distal end, such the guidewire element is suitable for use in the treatment of both partial and total occlusions.

### DESCRIPTION OF THE SPECIFIC EMBODIMENTS

Multilumen fluid delivery systems are provided. The subject multilumen fluid delivery systems include a multilumen aspiration catheter over a guidewire, which in many embodiments is a hollow guidewire. Also provided are kits that include various components of the subject systems. The subject multilumen fluid delivery systems and kits find use in a variety of different applications in which it is desired to flush a vascular site with at least one and preferably two different fluids, where particular applications of interest include the treatment of vascular lesions, and particularly coronary vascular lesions.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

### SYSTEMS

As summarized above, the present invention provides a multilumen catheter system. Specifically, the subject invention provides a system made up of an aspiration catheter element and a guidewire element that are designed to be used in concert to flush a vascular site with a treatment solution. Each of these elements is now described separately in greater detail.

### Aspiration Catheter

The aspiration catheter of the subject multilumen fluid delivery systems is a four-lumen catheter that includes a lumen employed for aspiration when the catheter is used in a system for flushing a vascular site with fluid. The aspiration catheter is characterized by having a proximal end and a distal end separated by a non-coaxial four-lumen elongated tube, where a five-port manifold is present at the proximal end. By non-coaxial multilumen tube, it is meant a tube that includes at least two lumens that are non-coaxial, i.e., they do not share a common axis. By elongated, it is meant that the distance between the proximal and distal ends is sufficient for the catheter to be inserted or introduced into the vascular system of a patient at a site remote from the vascular lesion that is to be treated through action of the distal end of the catheter, as is known in the art. Catheters intended for intravascular introduction will typically have a length in the range from about 50 cm to about 200 cm and an outer diameter in the range from about 1.0 Fr. to about 12.0 Fr., usually from about 1.0 Fr. to about 9.0 Fr. In the case of coronary catheters, the length is typically in the range from about 125 to about 200 cm, the diameter is often below about 8.0 Fr., more often below about 7.0 Fr., and most often in the range from about 2.0 Fr. to about 7.0 Fr. In certain embodiments, the elongated tubular element has a length of from about 80 to about 200 cm, usually from about 90 to about180 cm and more usually from about 90 to about140 cm.

By five-port manifold is meant a manifold that includes five ports (in addition to the attachment structure of the mannifold to the proximal end of the elongated tube of the catheter). At least four of the ports of the multiport manifold preferably have an attachment element for establishing a sealed fluid communication with the lumen of an external tubular element or analogous fluid conveying means, where this attachment structure is generally referred to herein as a luer-type connector or a luer-valve, where many different luer-type connectors or analogous attachment elements are known to those of skill in the art. In many embodiments, a luer valve is present at one or more ports of the multiport manifold, where the valve may be a male or female luer valve. Luer valves are disclosed in U.S. Patent Nos. 6,063,062; 6,039,302; 5,954,313; 5,947,954; 5,788,215; 5,775,671; 5,738,144; 5,549,651; 5,535,785; 5,474,544; 5,441,487; 5,372,143; 5,284,475. The fifth port has a valve capable of opening and closing around a tubular element, e.g., the guidewire element of the subject invention, as described in greater detail below, etc., to produce a sealed relationship with the guidewire element. In many embodiments, this sealing element is a Touhy-Borst valve or analogous structure, where Touhy-Borst valves are known to those of skill in the art and described in U.S. Pat. Nos.: 5,795,307 and 5,320,613.

Another common feature of the aspiration catheters of the subject invention is that the elongated multilumen tube is typically a polymeric extruded element, which is made up of one or more biocompatible polymers that have been extruded to produce the non-coaxial multilumen tube. Biocompatible polymers of interest include, but are not limited to: polyimide, polyamide, PBAX^{™}, polyethylene, polyisoprene, nylon and the like.

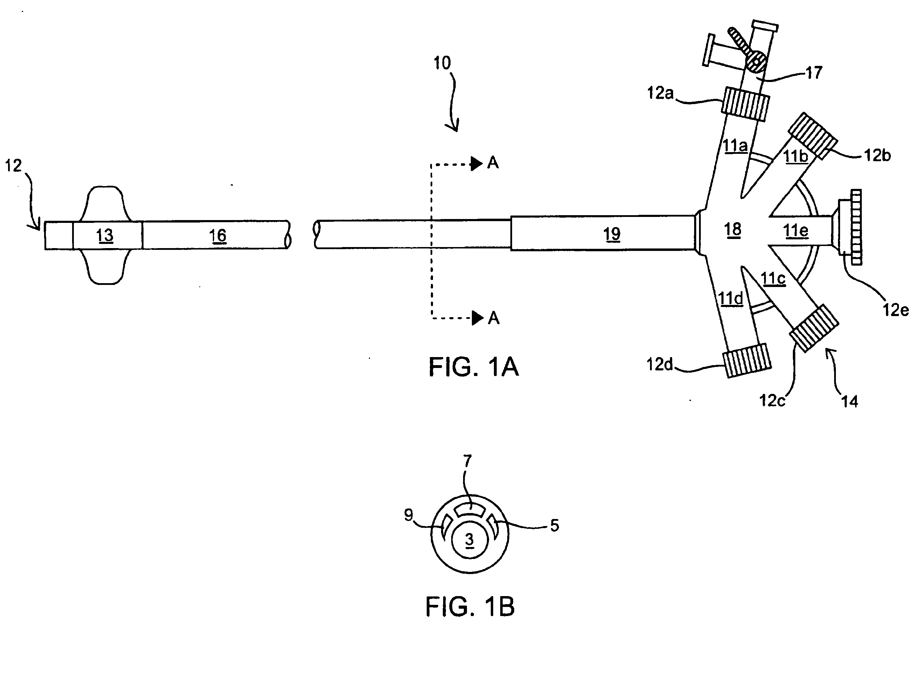

Figs. 1A and B provide a depiction of a representative aspiration catheter according to the subject invention. In Fig. 1A, aspiration catheter 10 has proximal end 14 and distal end 12 separated by elongated non-coaxial four-lumen tube 16. The length of elongated tube 16 should be long enough to provide for access of the distal end to the target vascular site upon introduction into the host, subject or patient at a remote site, and typically ranges from about 80 to about 200 cm, usually from about 90 to about 180 cm and more usually from about 90 to about 140 cm. The outer diameter of the elongate tubular member 16 may vary depending on the target vascular site for which it is designed to be used. In other words, the outer diameter of the aspiration catheter is selected so as to provide for access of the distal end of the catheter to the vascular site via the vascular system from the remote point of entry, where the outer diameter typically ranges from about 1.0 Fr. to about 12.0 Fr, usually from about 1.0 to about 9.0Fr. In the case of coronary catheters, the length is typically in the range from about 125 to about 200 cm, the diameter is often below about 8.0 Fr., more often below about 7.0 Fr., and most often in the range from about 2.0 Fr. to about 7.0 Fr.

The aspiration catheter is further **characterized in that** one of the four lumens, generally the larger of the four lumens, opens at the distal end, thereby providing a fluid entry site for fluid from the target vascular site to flow into the lumen. Figure 1B provides a cross-sectional view along Line A-A showing the four lumens of the four lumen tube 16. In Fig. 1B, this larger lumen is designated 3. The inner diameter of this lumen at the open distal end and along the entire length of the tube 16 is sufficient to house the guidewire element of the subject systems, as described in greater detail below, and remove fluid from the vascular site at the desired rate, e.g., a rate that provides for substantially isometric or isobaric pressure in the vascular site during treatment, through the resultant annular space. This larger lumen 3 is also known as the aspiration lumen. The inner diameter of the aspiration lumen 3, at least at its distal end and generally along the entire length of the aspiration catheter, is sufficient to provide for adequate flow into the lumen of the catheter, and sometimes ranges from about 0.20 to about 2.0, usually from about 0.25 to about 1.75 and more usually from about 0.35 to about 1.5 mm.

Also present at the distal end of the aspiration catheter 10 is a vessel occlusion means 13, where the vessel occlusion means is usually an inflatable balloon. The balloon 13 is one that is inflatable to a volume sufficient to substantially occlude the vessel in which the aspiration catheter is positioned, e.g., by pressing against the intimal surface of the vessel in which the aspiration catheter is positioned. The inflated balloon diameter generally ranges from about 2 to about 30 mm, usually from about 5 to about 20 mm. The inflated balloon length typically ranges from about 10 to about 30 mm, usually from about 15 to about 20 mm. The balloon is in fluid or gaseous communication with an inflation lumen 5 that is a second lumen of the four-lumen tube and runs the length of the aspiration catheter from the balloon to the proximal end, but does not open up at the distal end of the catheter. The inflation lumen typically has an inner diameter that ranges from about 0.1 to about 0.5 mm, usually from about 0.2 to about 0.4 mm.

The third lumen 9 of the four-lumen tube is typically employed for delivery of a dissolution fluid attenuating fluid to the target vascular site, e.g., for delivery of a buffer solution to the target vascular site. The cross-sectional area of this dissolution fluid delivery lumen 9 typically ranges from about 0.03 to about 0.8 mm², usually from about 0.03 to about 0.4 mm² and more usually from about 0.07 to about 0.2 mm².

The fourth lumen 7 of the four-lumen catheter is employed to deliver or convey a dissolution fluid. The inner diameter of lumen 7 typically ranges from about 0.2 to about 1.0 mm, usually from about 0.2 to about 0.7 mm and more usually from about 0.3 to about 0.4 mm, so as to provide for a cross-sectional area of about 0.03 to about 0.8 mm², usually from about 0.03 to about 0.4 mm² and more usually from about 0.07 to about 0.2 mm². The cross-sectional area of lumen 7 typically ranges from about 0.03 to about 0.8 mm², usually from about 0.03 to about 0.4 mm² and more usually from about 0.07 to about 0.2 mm².

While the configuration of the representative four-lumen aspiration catheter depicted in Figures 1A and B shows circular and crescent shaped lumens, other configurations are also possible, e.g., two circular lumens, to semicircular lumens, etc.

At the proximal end 14 of aspiration catheter 10 is five-port manifold 18. Five-port manifold 18 includes side or offset ports 11a, 11b, 11c and 11d as well as central port 11e. One of the offset ports, e.g., 11a is in fluid communication with the smaller lumen 5 and is attached to a balloon inflation means, e.g., a syringe filled with gas or fluid, during use. The second offset port, e.g., 11b, is in fluid communication the larger aspiration lumen 3 and is attached to a source of negative pressure, e.g., a vacuum, during use. The third offset port, 11c, is in fluid communication with a source or reservoir of dissolution fluid attenuating fluid and dissolution fluid delivery lumen 9. The fourth offset port 11d is in fluid communication with a source or reservoir of dissolution fluid and dissolution fluid delivery lumen 7. Offset ports 11a, 11b, 11c and 11d are further characterized in having or being in communication with luer valves 12a, 12b, 12c, and 12d respectively, for making connections with fluid conveyance means. In many embodiments, luer valves 12a, 12b, 12c and 12d are female luer valves. Central port 11e is in fluid communication with the aspiration lumen 3 and provides the point of access for the guidewire element, described in greater detail below. As such, in the manifold 18 of the aspiration catheter, two of the ports of the manifold, specifically the central port and one of the offset ports, are in fluid communication with the aspiration lumen 3. Central port 11e is characterized by the presence of Touhy-Borst valve 12e or an analogous structure. Touhy-Borst valves suitable for use in medical devices, e.g., catheters, are known in the art and described in U.S. Patent No. 5,320,613 and 5,795,307.

Also present on the catheter shown in Fig. 1A is a strain relief 19. The strain relief protects the proximal end of tube 16 from damage and gives strength to the transition between the tube and manifold 18. The strain relieve locally increases the stiffness of tube 16 to provide a moderated step-wise increase in stiffness from the relatively more flexible tube to the stiffer manifold member. The length of the strain relieve may vary from about 5 to 40 mm, but is usually 20 to 30 mm in length. Suitable materials for strain relief 18a include fluorinated ethylene-propylene or a similar medical grade polymer.

The embodiment shown in Fig. 1A may include three-way stop cocks attached to one or more of the offset ports. For example, in Fig. 1A, three-way stop cock 17 is positioned at the opening of offset port 11a. These elements provide for further control of fluid flow into the lumens of the device and/or the introduction of two separate fluids into the same lumen of the device, e.g., an imaging solution into the aspiration lumen and aspiration of fluid through the aspiration lumen, depending on the state of the three -way stop-cock.

For convenience during use of the aspiration catheter, each of the ports of the five-port manifold may be uniquely identified, e.g., color coded, so that it is readily apparent as to the element that the port should be connected during use of the device, e.g. vacuum source, balloon inflation means, etc. For example, one of the offset ports may have a yellow band, one may have a green band, one may have a blue band, one may have an orange band and one may have a red band, thereby uniquely identifying the ports of the four-port manifold.

### Guidewire Element

The second element of the subject fluid delivery systems is the guidewire element. Where the system is intended to treat a total vascular occlusion, the guidewire element may be any standard guidewire that can be placed inside of the aspiration lumen of the aspiration catheter component. Representative guidewires include, but are not limited to, those guidewires described in U.S. Patent Nos. 6,007,514; 5,980,471; 5,957,865; 5,938,609; 5,931,819; 5,916,178; 5,908,395; 5,902,254; 5,865,767; 5,827,201; 5,788,654; 5,772,609; 5,769,796; 5,755,695; 5,749,837; 5,682,897; 5,660,180; 5,636,642; 5,606,981; 5,599,492; 5,596,996; 5,558,093; 5,546,948; 5,520,189; 5,507,301; 5,497,782; D363,776; 5,460,187; 5,441,497; 5,437,288; 5,427,118; 5,421,349; 5,411,033; 5,409,015; 5,368,035; 5,341,818; 5,339,833; 5,313,967; 5,303,714; RE34,466; 5,265,622; 5,238,005; 5,184,621; 5,167,239; 5,147,317; 5,144,959; 5,111,829; 5,107,852; 5,095,915; 5,095,911 5,084,022; 5,069,226; 5,063,935; 4,966,163; 4,953,553; 4,875,489; 4,827,941; 4,811,743; 4,676,249; 4,534,363; 4,080,706 and 4,003,369.

The dimensions of the guidewire may vary, so long as the guidewire can slidably fit inside of the aspiration lumen.

In certain applications, the guidewire element is a hollow guidewire, where such guidewires include, but are not limited to, those guidewires described in U.S. Patent Nos.: 5,569,197; 6,068,623; 6,217,567; and 6,059,767.

In certain embodiments, the hollow guidewire includes a vascular occlusion element at its distal end. Hollow guidewires with vascular occlusion elements located at their distal end are also known to those of skill in the art, see e.g., U.S. Patent Nos. 6,050,972; 5,833,650; and 5,167,239.

Hollow guidewires with vascular occlusion elements at their distal end are preferred in certain embodiments, as these guidewires can be used for the treatment of both partial and total occlusions. As such, systems that include such guidewires need not include three distinct elements to be used in the treatment of either partial or total occlusions, since the same guidewire/delivery element can be used for the both the total and partial occlusions.

The guidewire elements of the subject invention have sufficient structural integrity, or "pushability," to permit the aspiration catheter to be advanced through vasculature to distal arterial locations without buckling or undesirable bending of the guidewire element. It is also desirable that the guidewire element have the ability to transmit torque, such as in those embodiments where it may be desirable to rotate the guidewire after insertion into a patient. A variety of biocompatible materials, known by those of skill in the art to possess these properties and to be suitable for catheter manufacture, may be used to fashion the guidewire. For example, the guidewire element may be made of stainless steel, or may be made of polymeric materials such as nylon, polyamide, polyimide, polyethylenes, or combinations thereof, shape memory materials, such as alloys of titanium and nickel, commonly referred to as nitinol.

The production and manufacture of suitable hollow guidewires that may be used as the delivery elements of the subject systems are known to those of skill in the art. See e.g., U.S. Patent Nos. 6,217,567 and 6,068,623.

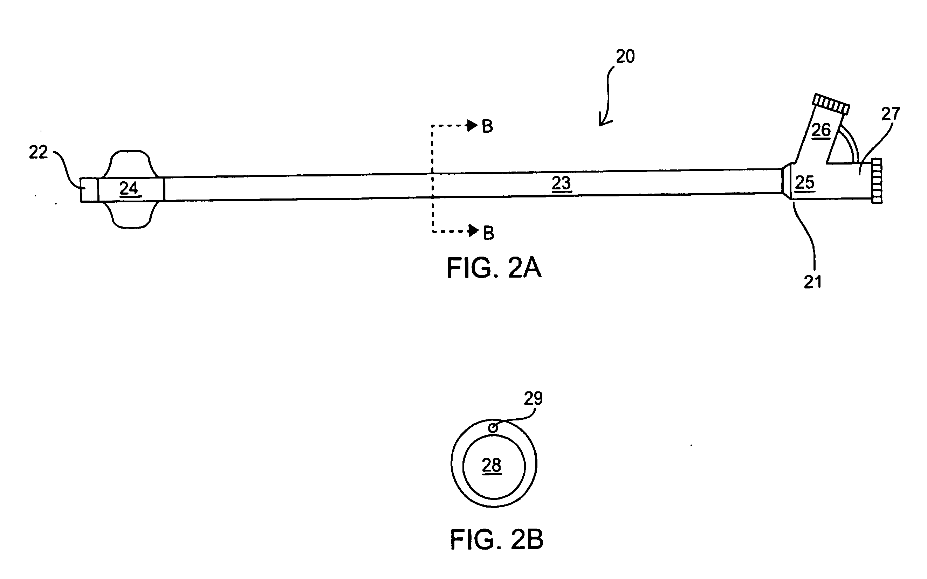

Figs 2A and 2B provide views of a representative hollow guidewire element that may be employed in systems of the subject invention. Hollow guidewire 20 includes proximal and distal ends, 21 and 22, respectively, separated by elongated tubular structure 23, which has dimensions and physical attributes that allow it to be employed as a guidewire. Located at the distal end of the guidewire is occlusive balloon 24. At the proximal end 21 is two port manifold 25 which includes an offset port 26, for connecting a balloon inflation element to the balloon inflation lumen of the guidewire and central port 27 for connecting the central lumen of the hollow guidewire to a source of fluid, e.g., a perfusate fluid. Figure 2B provides a cross-sectional view of guidewire 20 along line B-B. In Figure 2B, central fluid delivery lumen 28 and balloon inflation lumen 29 are non-coaxially positioned inside of tubular member 23.

The subject systems, as described above, are useful in flushing a vascular site with two different fluids. By flushing a vascular site is meant introducing fluid into and removing fluid from a vascular site at substantially the same time such that the vascular site remains substantially stable in terms of pressure, e.g., is isobaric, where the pressure changes that occur in the vascular site do not exceed in magnitude a value of about 50 mm Hg and usually do not exceed about 30 mm Hg. Accordingly, maximum pressure will typically remain below about 400 mm Hg, preferably about 100 mm Hg.

During use, the subject systems are characterized by having an aspiration catheter and a guidewire element of the subject invention in a nested configuration, such that the guidewire element is slidably positioned inside the aspiration lumen of the aspiration catheter. Because the central manifold port of the aspiration catheter may have a Touhy-Borst valve, a fluid seal can be produced at the interface between the aspiration manifold central port and the outer surface of the inserted guidewire element when inserted into the aspiration lumen of the aspiration catheter.

In using the systems of the subject invention, one of the offset ports of the aspiration manifold is often in fluid communication with a syringe balloon inflation means, another of the offset ports of the aspiration manifold is in fluid communication with a vacuum source to provide the force for removing fluid from the vascular site via the aspiration lumen, a third offset port is in fluid communication with the a source of dissolution fluid, e.g., an ENDOFLATOR®, etc.; and a fourth offset port is in fluid communication with a source of dissolution fluid attenuating fluid e.g., an ENDoFLATOR®, etc.

The aspiration catheter is positioned over the guidewire element, e.g., by sliding the aspiration catheter over the guidewire element. Where the system is used for total occlusion, treatment the guidewire element may be a solid guidewire. Alternatively, the guidewire may be a hollow guidewire, where the lumen of the hollow guidewire may be employed to deliver fluid to the vascular site, e.g., dissolution fluid or dissolution fluid attenuating fluid. The guidewire may also be a hollow guidewire with an occlusive element at its distal end, e.g., as depicted in Figs 2A and 2B, so that the system may be employed for both partial and total occlusion treatment, without having to employ a different guidewire element depending on the nature of the target occlusion. Where the system is employed for a partial occlusion, that aspiration catheter is positioned over a guidewire element that includes an occlusive element at its distal end. In certain embodiments, the guidewire element is a hollow guidewire element, such as that shown in Figures 2A and 2B, so that a perfusate may be delivered to the vascular system beyond the occlusive element.

### METHODS

The subject systems find use in applications where it is desired to simultaneously flush a vascular target site with two different fluids. As mentioned above, by flush it is meant that the fluid is introduced into the vascular site and removed from the vascular site in a manner such that the vascular site remains at substantially constant pressure. However, the pressure throughout the treatment site is predicted to be, on average, lower than the backbleed pressure, i.e., the blood pressure on the proximal side of the aspiration catheter balloon. While the subject systems can, in principle, be employed to flush a vascular site with any two fluids, they are particularly suited for use in applications where chemical tissue ablation at a target vascular site is desired. As such, the subject systems find particular use in the treatment of vascular lesions or obstructions, where the target lesions or obstructions may be organic, inorganic or composite structures of both organic and inorganic components. In such example, the systems are used to flush the target vascular site, and therefore the lesion or obstruction located therein, with a dissolution fluid and a dissolution fluid attenuating fluid.

In these examples of the subject methods, the first step is generally to provide for an entry site for the catheter into the vascular system of the host. Entry is typically provided by placement of an introducer sheath at a convenient location, e.g. leg etc., as is known in the art. The guidewire element of the subject systems is then inserted through the entry sheath and its distal end is placed at the target vascular site. The aspiration catheter is then moved over the guidewire, where the guidewire generally passes through the central lumen, so that the distal end of the aspiration catheter reaches the target vascular site. In many embodiments, the distal end of the guidewire element will extend some distance beyond the distal end of the aspiration catheter, where this distance typically does not exceed about 20 cm and usually does not exceed about 5 mm.

Upon positioning of the catheter system as described above and deployment of any vascular occlusion means, the dissolution fluid and dissolution fluid attenuating fluid are introduced into the vascular site via the appropriate lumens inside the aspiration catheter and fluid is removed from the vascular site via the aspiration lumen of the aspiration catheter, and specifically through the annular space present in the system bordered by the inner wall of the aspiration lumen and the outer wall of the guidewire. The nature of the dissolution fluid and the dissolution fluid attenuating fluid necessarily depends on the nature of the target lesion to be treated. For example, for organic matter comprising lesions, organic matter dissolution fluids (and their companion attenuating fluids) are of interest, such as those described in U.S. Application Patent No. 09/528,576. In other examples where the target lesion comprises inorganic matter, acidic dissolution solutions and their companion buffer attenuating fluids are of interest, such as those described in WO 00/03651. See e.g., WO 00/03651; WO 01/13985; WO 01/15767; WO 01/39783; WO 01/70320; and WO 02/15958.

In many examples, the dissolution fluid employed in the subject methods is an inorganic matter dissolution solution. In many of these examples the inorganic matter dissolution fluid is an acidic dissolution fluid. A variety of different types of acidic dissolution solutions may be employed in the subject methods. The acidic treatment solutions that find use in the subject methods generally have a pH of less than about 6.5, where the pH is usually less than about 4.0 and more usually less than about 3.0. In many examples, the pH ranges from 0 to 2, and usually 0 to 1. The acidic treatment solution can include a number of different types of acids, where the acids may or may not include a hydrocarbon moiety, i.e., a hydrogen bonded directly to a carbon atom. Suitable acids that lack a hydrocarbon moiety include halogen acids, oxy acids and mixtures thereof, where specific acids of interest of this type include, but are not limited to, hydrochloric, nitric, sulfuric, phosphoric, hydroboric, hydrobromic, carbonic and hydroiotic acids. For such acids, the acid can be a concentrated acid, or can be diluted. Upon dilution, the concentration of an inorganic acid will generally be from about 10 N to about 0.01 N, preferably between 5 N to 0.1 N. Also of interest are acids that include a hydrocarbon moiety, where such acids include, but are not limited to, any organic acid of one to six (C₁ to C₆) carbons in length. Organic acids of this type include, but are not limited to, formic, acetic, propionic, maleic, butanoic, valeric, hexanoic, phenolic, cyclopentanecarboxylic, benzoic, and the like. For an organic acid, the acid can be in concentrated form, or can be diluted. The acidic treatment solution can be composed of either a monobasic or a polybasic acid. Acids are "monobasic" when they have only one replaceable hydrogen atom and yield only one series of salts (*e*.*g*., HCl). Acids are "polybasic" when they contain two or more hydrogen atoms which may be neutralized by alkalies and replaced by organic radicals.

In many examples the acid solution is hypertonic, by which is meant that the osmolarity of the solution is greater than that of whole blood, i.e. the osomolarity is greater than 300 mosmol. The solution may be rendered hypertonic by including any convenient component or components in the solution that provide for the desired elevated osmolarity.

Any convenient agent that is capable of increasing the osmolarity of the solution may be employed, where suitable agents include salts, sugars, and the like. In many examples, the agent that is employed to render the solution H hypertonic is one or more, usually no more than three, and more usually no more than two, different salts. Generally, the salt concentration in these examples of the solution is at least about 100 mosmol, usually at least about 200 mosmol and more usually at least about 300 mosmol, where the concentration may be as high as 3000 mosmol or higher, depending on the particular salt being employed to render the solution hypertonic, where the solution may be saturated with respect to the salt in certain examples. Salts that may be present in the subject solutions include: NaCl, MgCl₂, Ringers, etc. where NaCl is preferred in many examples.

Of particular interest in many examples is the use of a hydrogen chloride solution. In hydrogen chloride solutions, the concentration of HCl in the solution ranges from about 0.001 to 1.0 N, usually from about 0.01 to 1.0 N and more usually from about 0.1 to 1.0 N. In many examples, the hydrogen chloride solution will further include one or more salts which make the solution hypertonic, as described above. In certain examples, the salt is NaCl, where the concentration of NaCl in the solution is at least 0.05 M, usually at least 0.10 M, and more usually at least 0.15 M, where the concentration may be as high as 0.25 M or higher. In certain examples, the solution will be saturated with NaCl. H

Of particular interest are aqueous hydrogen chloride solutions that consist of water, hydrogen chloride and NaCl. The concentration of hydrogen chloride in these solutions of particular interest ranges from about 0.01 to 1.0 N, usually from about 0.05 to 0.5 N and more usually from about 0.075 to .25 N. The concentration of NaCl in these solutions of particular interest ranges from about 0.05 to 0.25 M, usually from about 0.05 to 10 M.

In certain examples, one or more of the delivery fluids is present at a temperature that is less than room temperature. For example, in certain examples, the one or more treatment solutions, as described above, is present at a temperature ranging from about 0 to about 20 °C, sometimes from about 0 to 15°C, e.g., from about 0 to 10°C. Such examples include applications where it is desired to limit restinosis by employing reduced temperature, e.g., cold, solutions.

The target vascular site is flushed with the dissolution and dissolution fluid attenuating fluids for a period of time sufficient to result in the desired amount of treatment, e.g., target lesion size reduction, enhancement or establishment of fluid flow through the target site, etc. Following the desired amount of treatment, the system is removed from the host. More specific detail regarding the methods in which the subject systems find use can be found in U.S. Patent No. 09/528,576; and publication no. WO 00/03651.

In certain examples, external energy is applied to the target aortic valve H to promote mechanical break-up of the calcified deposits into particles or debris that can be easily removed from the vascular site. Any means of applying external energy to the aortic valve may be employed. As such, jets or other such means the device which are capable of providing varying external forces to the target deposits cause the target deposit to break up or disrupt may be employed. Of particular interest in many examples is the use of sonic energy, e.g., infrasound, ultrasound, etc. The sonic energy, e.g., ultrasound, can be applied during the entire time of contact of the cardiovascular tissue with the acidic treatment solution, or it can be applied for only part of the treatment period. In one example, the sonic energy is applied for several short periods of time while the dissolution treatment solution is contacted with the target occlusion. Sonic energy elements that are suitable for use with the subject methods are known and readily available to those of skill in the art. For example, U.S. Patent Nos.: 5,695,460; 5,997,497; 6,047,700; 6,083,573; 6,113,570; and 6,308,714; as well as the United States Patents cited therein; all describe various ultrasound technologies for delivering ultrasound to a physiological, including cardiovascular, site, where such technologies include transdermal delivery technologies. Another means that may be employed to apply external energy to the lesion during the dissolution process is to use a mechanical means of applying external energy. Mechanical means of interest include moving structures, e.g. rotating wires, guidewires, which physically contact the target lesion and thereby apply physical external energy to the target lesion.

In certain examples, as described above, the local environment of the target lesion is rendered substantially bloodless prior to introduction of the acidic dissolution fluid. In these embodiments, the isolation system is deployed to physically isolate the local environment from the remainder of the circulatory system and then the local environment is flushed with a physiologically acceptable solution, such that substantially all of the blood present in the solution is removed. Typically, a washing solution will be employed in this step of rendering the local environment bloodless. Examples of washing solutions that may find use in these embodiments include: water for injection, saline solutions, e.g. Ringer's, phosphate buffered saline, or other physiologically acceptable solutions. The washing solution may include an anti-clotting factor in many examples, where anticlotting factors of interest include heparin and the like. The washing solution can also contain chelating agents.

In addition, it may be convenient to monitor or visualize the vascular site prior to or during treatment. A variety of suitable monitoring means are known to those of skill in the art. Any convenient means of invasive or noninvasive detection and/or quantification may be employed. Such means include plain film roentgenography, coronary arteriography, fluoroscopy, including digital subtraction fluoroscopy, cinefluorography, conventional, helical and electron beam computed tomography, intravascular ultrasound (IVUS), magnetic resonance imaging, transthoracic and transesophageal echocardiography, rapid CT scanning, antioscopy and the like. Any of these means can be used to monitor the vascular site before, during or after contact with the dissolution fluid.

In many examples, an imaging agent is employed, where the imaging agent may or may not be present in the acidic dissolution solution. Imaging agents of particular interest include: non-ionic imaging agents, e.g. CONRAY^{™}, OXILAN^{™}, and the like.

In certain examples as mentioned above, the guidewire element is a hollow guidewire. In these examples, the guidewire may be employed for a number of additional functions. For example, where a hollow guidewire is employed in the total occlusion system, the guidewire may be employed to convey additional dissolution fluid to the target site. In other examples where the hollow guidewire is employed in the partial occlusion system, it may be employed to convey a perfusate, such as an oxygen bearing medium, to a region beyond the distal balloon and isolated environment bordered thereby. Perfusates of interest include, but are not limited to: (1) the oxygen gas supersaturated fluids as described in U.S. Patent Nos. 6,315,754; 5,693,017; 5,797,874; and the like.

While the methods are suitable for use in the treatment of any type of vascular lesion, the methods are particularly suited for treatment of coronary vascular lesions, specifically coronary vascular calcified lesions, such as lesions that might be found in coronary arteries, which lesions may be total or partial occlusions, as described above. By using the subject devices suitably dimensioned to be positioned inside of the coronary vascular structure to be treated, coronary vascular lesions can readily be treated according to the subject invention. The subject methods may be employed in conjunction with other coronary vascular treatments, e.g., stent deployment, etc.

### KITS

Also provided are kits for use in flushing a vascular site with two fluids. The subject kits at least include the components of a partial occlusion or total occlusion catheter system according to the subject invention. As such, the kits include an aspiration catheter and at least one guidewire element. In certain examples, the kits include at least two different guidewire elements, one that is suitable for use in total occlusion systems and one that is suitable for use in partial occlusion systems. In yet other examples, the kits include a single guidewire element that is suitable for use in both partial and total occlusion systems, e.g., such as the one shown in Figures 2A and 2B.

The subject kits may also include a dissolution fluid and/or dissolution fluid attenuating fluid, or components/precursors thereof, where representative dissolution fluids and dissolution fluid attenuating fluids are disclosed above. The dissolution fluids and dissolution fluid attenuating fluids or component(s) thereof are present in the kit in a suitable container, e.g., a bottle, pouch, fluid filled an ENDoFLATOR®, etc. which is capable of serving as a storage vessel for the this component of the kit and, preferably, capable of preserving the sterility of this component of the kit, as this component of the kit is preferably sterile, e.g., medical grade.

The kits may also include a number of different optional components, which components may find use in methods in which the subject kit components are employed, e.g., dilators for use in creating entries into the vascular system of the host. Additional optional components that may be present in kits include various fluids and solutions in addition to the dissolution fluid and dissolution fluid attenuating fluid described above. Additional fluids that may be present include: organic matter dissolution fluids, wash or rinsing fluids, imaging agent fluid mediums that include an imaging agent, such as a non-ionic imaging agents, e.g., CONRAY^{™}, OXILAN^{™}, fluids containing one or more pharmacological agents, e.g., agents that promote healing, reduce inflammation, and the like; etc. Other components that may be present in the subject kits include one or more additional components and accessories for use with the fluid delivery means present in the kit, including tubing for connecting the various catheter components with fluid reservoirs, syringes, pumps, etc., connectors, stop-cocks, dilators, insertion sheaths, vacuum regulators, negative pressure or vacuum generators/sources, luer valve adapters, etc.

In addition to above mentioned components, the subject kits typically further include instructions for using the components of the kit to flush a vascular site with two different fluids, e.g., to flush a vascular site with a dissolution fluid and a dissolution fluid attenuating fluid. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other examples, the instructions are present as an electronic storage data file present on a suitable computer readable storage medium, e.g. CD-ROM, diskette, etc. In yet other examples, the actual instructions are not present in the kit, but means for obtaining the instructions from a remote source, e.g. via the internet, are provided. An example of this is a kit that includes a web address where the instructions can be viewed and/or from which the instructions can be downloaded. As with the instructions, this means for obtaining the instructions is recorded on a suitable substrate.

It is evident from the above discussion that the catheters and systems that are made up of the same provide a reliable and convenient way to flush a target vascular site with two different fluids. Because the catheter devices have a design that allows them to be produced via extrusion technology using polymeric materials, they may be economically produced and the number of different working elements is kept to a minimum. In addition, the catheter design allows the catheters to be scaled to treat a wide variety of different type of vascular lesions, including coronary vascular lesions. In view of these factors and such others discussed above, the subject invention represents a significant contribution to the art.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

## Claims

1. A multilumen catheter (10) comprising:
(a) a proximal end (14);
(b) a distal end (12); and
(c) a five-port manifold (18) located at said proximal end, wherein:
(i) said five-port manifold comprises at least four ports (11 a,b,c,d) having luer-type connectors (12 a,b,c,d) and a fifth port (11e) comprising a sealing element (12e) for producing a sealing engagement around a tubular element inserted therethrough; and
(ii) said proximal and distal ends are separated by a non-coaxial four-lumen tube (16),
**characterised in that**
one of said ports (11c) of said five-port manifold of said multilumen catheter is in fluid communication with a dissolution solution attenuating solution fluid reservoir, and
one of said ports (11d) of said five-port manifold of said multilumen catheter is in fluid communication with a dissolution solution fluid reservoir.

2. A system for flushing a vascular site with fluid, said system comprising:
(I) a multilumen catheter (10) according to claim 1; and
(II) a guidewire (20).

3. A system for flushing a vascular site with a fluid, according to claim 2, wherein the guidewire is inserted through said fifth port (11^{e}) and present in one of said four lumens (3,5,7,9).

4. The system according to Claim 2 or 3, wherein said guidewire (20) is a hollow guidewire comprising at least one lumen (28,29).

5. The system according to Claim 4, wherein said hollow guidewire includes a vascular occlusion element (24) at a distal end (22).

6. The system according to any of Claims 3 to 5, wherein said sealing element (12^{e}) of said central port (11e) produces a sealing engagement with said guidewire (20) when inserted therethrough.

7. The system according to any of Claims 3 to 6, wherein one of said ports (11 a,b,c,d,e) of said five-port manifold (18) of said multilumen catheter (10) is in fluid communication with a negative pressure source.

## Patentansprüche

1. Mehrlumen-Katheter (10), welcher aufweist:
(a) ein nahes Ende (14);
(b) ein entferntes Ende (12); und
(c) einen Fünffach-Verteiler (18), der an dem nahen Ende angeordnet ist, wobei:
(i) der Fünffach-Verteiler (18) zumindest vier Anschlüsse (11 a, b, c, d) mit Luer-Typ-Anschlusselementen (12 a, b, c, d) und einen fünften Anschluss (11 e) mit einem Abdichtungselement (12 e) zum Herstellen einer Abdichtungsverbindung um ein Schlauchelement, das durch das Abdichtungselement (12 e) eingeführt wird, aufweist; und
(ii) das nahe Ende und das entfernte Ende durch einen nicht koaxialen Vier-Lumen-Schlauch (16) voneinander getrennt sind,
**dadurch gekennzeichnet, dass**
einer der Anschlüsse (11 c) des Fünffach-Verteilers des Mehrlumen-Katheters in Fluid-Verbindung mit einem Auflösungslösung-Verdünnungslösung-Flüssigkeitsbehälter steht, und
einer der Anschlüsse (11 d) des Fünffach-Verteilers des Mehrlumen-Katheters in Fluid-Verbindung mit einem Auflösungslösung-Flüssigkeitsbehälter steht.

2. System zum Durchspülen einer GeLäßstelle mit einer Flüssigkeit, welches aufweist:
(I) ein Mehrlumen-Katheter (10) gemäß Anspruch 1; und
(II) einen Führungsdraht (20).

3. System zum Durchspülen einer Gefäßstelle mit Flüssigkeit gemäß Anspruch 2,
wobei der Führungsdraht durch den fünften Anschluss (11 e) eingeführt und in einem von vier Lumen (3, 5, 7, 9) angeordnet wird.

4. System gemäß einem der Ansprüche 2 oder 3,
wobei der Führungsdraht (20) ein hohler Führungsdraht ist, der zumindest ein Lumen (28, 29) aufweist.

5. System gemäß Anspruch 4,
wobei der Führungs-Hohldraht ein Gefäßverschluss-Element (24) an einem entfernten Ende (22) aufweist.

6. System gemäß einem der Ansprüche 3 bis 5,
wobei das Abdichtungselement (12 e) des mittleren Anschlusses (11 e) eine Abdichtungsverbindung mit dem Führungsdraht (20) herstellt, wenn der Führungsdraht (20) durch das Abdichtungselement (12e) eingeführt wird.

7. System gemäß einem der Ansprüche 3 bis 6,
wobei einer der Anschlüsse (11 a, b, c, d, e) des Fünffach-Verteilers (18) des Mehrlumen-Katheters (10) in Fluid-Verbindung mit einer Unterdruckquelle steht.

## Revendications

1. Cathéter à plusieurs lumières (10) comprenant :
(a) une extrémité proximale (14) ;
(b) une extrémité distale (12) ; et
(c) un collecteur à cinq orifices (18) situé au niveau de ladite extrémité proximale, dans lequel :
(i) ledit collecteur à cinq orifices comprend au moins quatre orifices (11 a, b, c, d) ayant des connecteurs de type Luer (12 a, b, c, d) et un cinquième orifice (11e) comprenant un élément d'étanchéité (12e) pour produire une mise en prise étanche autour d'un élément tubulaire inséré à travers celui-ci ; et
(ii) lesdites extrémités proximale et distale sont séparées par un tube non coaxial à quatre lumières (16),
**caractérisé en ce que :**
l'un desdits orifices (11c) dudit collecteur à cinq orifices dudit cathéter à plusieurs lumières est en communication de fluide avec un réservoir de fluide de solution atténuant la solution de dissolution, et
l'un desdits orifices (11d) dudit collecteur à cinq orifices dudit cathéter à plusieurs lumières est en communication de fluide avec un réservoir de fluide de solution de dissolution.

2. Système pour rincer un site vasculaire avec du fluide, ledit système comprenant :
(I) un cathéter à plusieurs lumières (10) selon la revendication 1 ; et
(II) un fil guide (20).

3. Système pour rincer un site vasculaire avec un fluide, selon la revendication 2, dans lequel le fil guide est inséré à travers ledit cinquième orifice (11e) et est prévent dans l'une desdites quatre lumières (3, 5, 7, 9).

4. Système selon la revendication 2 ou 3, dans lequel ledit fil guide (20) est un fil guide creux comprenant au moins une lumière (28, 29).

5. Système selon la revendication 4, dans lequel ledit fil guide creux comprend un élément d'occlusion vasculaire (24) au niveau d'une extrémité distale (22).

6. Système selon l'une quelconque des revendications 3 à 5, dans lequel ledit élément d'étanchéité (12e) dudit orifice central (11e) produit une mise en prise étanche avec ledit fil guide (20) lorsqu'il est inséré à travers celui-ci.

7. Système selon l'une quelconque des revendications 3 à 6, dans lequel l'un desdits orifices (11 a, b, c, d, e) dudit collecteur à cinq orifices (18) dudit cathéter à plusieurs lumières (10) est en communication de fluide avec une source de pression négative.
